# EUROPEAN PATENT APPLICATION

(11) **EP 1 223 155 A1**
(43) Date of publication of application: **17.07.2002**
(21) Application number: 01200120.2
(22) Date of filing: 15.01.2001
(51) Int. Cl.: C07C 45/50, C07C 51/235, C07C 67/39, C07C 67/36, C07C 227/08, C07C 41/54, C07C 67/38

(54) **Process for the preparation of a caprolactam precursor from butadiene**

(71) Applicant: DSM N.V., 6411 TE Heerlen (NL)
(72) Inventor: Sielcken, Otto Erik, 6136 BG Sittard (NL); Smits, Hubertus Adrianus, 6212 GC Maastricht (NL); Spronken, Josephus Marie Hubertus, 6241 BK Bunde (NL); Wolters, Henricus Franciscus Wilhelmus, 6101 BT Echt (NL); Boogers, Jeroen Antonius Franciscus, 6224 JK Maastricht (NL); Gelling, Onko Jan, 6129 HV Urmond (NL)

(57) **Abstract**

The invention relates to a process for the preparation of a caprolactam precursor starting from butadiene, whereby the process comprises a step in which butadiene is hydroformylated into a mixture of pentenals in the presence of carbon monoxide, hydrogen and a hydroformylation catalyst. The invention also relates to a process in which butadiene is hydroformylated in the presence of an alcohol resulting in a reaction mixture comprising an acetal of pentenals. The invention further relates to the preparation of methyl-3-pentenoate by oxidizing a pentenal and/or an acetal of pentenal containing reaction mixture.

## Description

The invention relates to a process for the preparation of a caprolactam precursor from butadiene.

Such a process is described in WO-A-9702091. This publication discloses the reaction of butadiene with carbon monoxide and water to 3-pentenoic acid in the presence of hydrogen iodide and a catalyst system comprising iodine, rhodium and an activated carbon support.

A disadvantage of this process is that the use of iodide containing catalysts may result in corrosion of the equipment.

The object of the invention is a process in which the above mentioned disadvantage does not occur or occurs to a lesser degree.

This object is achieved in that the process comprises a step in which butadiene is hydroformylated into a mixture of pentenals in the presence of carbon monoxide, hydrogen and a hydroformylation catalyst.

It has been found that with this process no or less corrosion of the equipment occurs. An advantage of the process of the invention is that it is not necessary to use pure carbon monoxide. Carbonylation processes as for example described in WO-A-9709201 require pure carbon monoxide. Carbon monoxide is usually prepared starting from synthesis gas. The derivation of carbon monoxide from synthesis gas is cumbersome because several separation steps, including the separation of carbon monoxide and hydrogen, are needed. A mixture of carbon monoxide and hydrogen is obtained as an intermediate in the preparation of carbon monoxide starting from synthesis gas, which mixture can be used in the process of the invention. A further advantage is that hydrofomylation of butadiene, compared with carbonylation of butadiene, requires lower reaction temperatures (≤100 °C) resulting in higher catalyst stability.

Several attempts have been made in the prior art for preparing methyl-3-pentenoate by reacting butadiene with carbon monoxide and methanol in the presence of a carbonylation catalyst. Although some of the disclosed catalyst systems are less corrosive, compared with the catalyst system as disclosed in WO-A-9702091, a stable catalyst system which is also economically attractive is never disclosed. An additional advantage of the process of the present invention is that compounds formed in situ from butadiene having a boiling point near the boiling point of methyl-3-pentenoate, like for example vinylcyclohexene and octatriene, can be separated from the reaction mixture prior to methyl-3-pentenoate synthesis. Vinylcyclohexene and octatriene are undesired by-products as they result in undesired side-reactions and/or are potential catalyst poisons in subsequent catalysed reactions of methyl-3-pentenoate. Still another advantage of the process of the invention is that the boiling points of pentenals are lower than the boiling points of methyl pentenoates. This is advantageous as the separation of the catalyst from the product mixture can be performed at a lower temperature resulting in inter alia a higher catalyst stability during separation.

In the process according to the invention butadiene is reacted with carbon monoxide, hydrogen and a hydroformylation catalyst in a liquid medium. The hydroformylation catalyst may be a solubilized metal-ligand complex catalyst. A preferred hydroformylation catalyst is a rhodium-organophosphorous ligand complex catalyst. Exemplary organophosphorous ligands are organophosphines, like for example monodentate and bidentate phosphines, and organophosphites, like for example monodentate and bidentate phosphites. Hydroformylation of butadiene using a rhodium-phosphine or rhodium-phosphite complex catalyst is for example described in WO-A-9740002, EP-A-577042, US-A-5312996, US-A-5817883, US-A-5821389, EP-A-872483, US-A-5892127 and US-A-5886237, the complete disclosures of which are incorporated as references. US-A-5710344 discloses the use of a complex of rhodium and bidentate phosphorous ligands containing four P-C bonds and two P-O bonds (so called phosphinites) . These publications do not mention or suggest to prepare a caprolactam precursor via pentenals or derivatives thereof.

Rhodium may be applied in any one of a variety of forms. The manner in which rhodium is introduced into the reaction mixture is not critical. As a rule, the catalytically active complexes based on a rhodium precursor such as for example Rh(CO)₂(acac) (acac=acetylacetonate), Rh₂O₃, Rh₄(CO)₁₂, Rh₆(CO)₁₆, Rh(NO₃)₂, Rh(Oac)₂ (Oac=acetate) are formed in the reaction mixture.

The rhodium concentration in the reaction mixture is not specially limited, but is optionally selected so that favorable results can be obtained with respect to catalyst activity and process economics. In general, the concentration of rhodium in the reaction mixture is between 10 and 10000 ppm, calculated as free metal.

The molar ratio of organophosphorous ligands to rhodium is not specially limited. Generally, this ratio is from about 0.5:1 or less to about 1000:1 or greater.

The hydroformylation is carried out at a temperature of between room temperature and 200 °C, preferably from 50 to 120 °C, and most preferred from 90 to 100 °C.

The hydroformylation is carried out at a pressure of between atmospheric pressure (0.1 MPa) to 20 MPa, preferably from 3 to 10 MPa. The pressure is generally equal to the combined hydrogen, carbon monoxide and butadiene partial pressures. The molar ratio hydrogen:carbon monoxide can be varied within wide limits and is generally between 10:1 and 1:10, preferably between 6:1 and 1:6 and most preferably betweem 2:1 and 1:2.

The hydroformylation reaction is preferably conducted in the presence of an organic solvent for the catalyst system and optionally free ligand. Suitable solvents include those commonly employed in known metal catalysed hydroformylation reactions. The solvent may be a mixture of reactants, such as the starting unsaturated compound, the aldehyde product and/or by-products. Suitable solvents include saturated hydrocarbons such as kerosene, mineral oil or cyclohexane, ethers such as diphenyl ether, tetrahydrofuran or a polyglycol, ketones such as methyl ethyl ketone and cyclohexanone, nitriles such as acetonitrile, valeronitrile, and benzonitrile, aromatics such as toluene, benzene and xylene, esters such as dimethylformamide, and sulfones such as tetramethylensulfone.

The pentenals which are formed during hydroformylation of butadiene will actually be a mixture of 2-, 3- and 4-pentenal. The 2- and 3-pentenal are present in their cis and trans configuration.
3-Pentenal is represented by the following formula:

CH₃-CH=CH-CH₂-CH=O (I)

It has been found that the pentenals should be prepared under strictly neutral conditions. With neutral conditions is meant neither acidic nor basic conditions. It has also been found that a pentenal containing mixture is preferably stored at strictly neutral conditions. Performing the preparation and/or storage under acidic or basic conditions results in undesired side-reactions, like for example aldol condensation and isomerisation, resulting in the formation of undesired by-products.

The hydroformylation reaction can be carried out batchwise, semi-continuously or continuously.

In a preferred embodiment of the invention, the hydroformylation is performed in the presence of an alcohol resulting in a reaction mixture comprising an acetal of pentenals. Hydroformylation of butadiene in the presence of an alcohol and a rhodium-phosphine complex catalyst is for example described in EP-A-94748, the complete disclosure of which is incorporated as reference. This disclosure do not mention or suggest to prepare a caprolactam precursor via acetals of pentenal. It has been found that the acetals of pentenal are easier to separate and/or to purify compared to the corresponding pentenals. It has also been found that the acetals of pentenal are less sensitive for the formation of heavy by-products, for example formed through aldol condensation. Performing the hydroformylation in the presence of an alcohol will be referred to as acetalisation. Examples of suitable alcohols are monohydric alcohols and diols. Examples of monohydric alcohols are methanol, ethanol, propanol, isopropanol, n-butanol, tert-butanol, pentanol, cyclohexanol and phenol. Examples of suitable diols are ethylene glycol, 2,3-butanediol, 2,3-dimethyl-2,3-butanediol and 2,4-pentanediol. The molar ratio of alcohol to butadiene is suitably in the range of 1:1 to 100:1, preferably larger than 2:1 and most preferably larger than 3:1. The larger the alcohol excess, the more complete the conversion of the acetalisation. The acetalisation is preferably performed in the presence of an acidic acetalisation catalyst. Examples of suitable acetalisation catalysts are sulphuric acid, a sulphonic acid, phosphoric, phosphorous acid, formic acid and acetic acid. Preferred acids are sulphuric acid and sulphonic acids, like for example paratoluene sulphonic acid. The concentration of the acetalisation catalyst lies as a rule between 0.05 and 5 g acid per kg reaction mixture. An acidic ion exchange resin is also suitable as acetalisation catalyst. An example of a suitable acidic ion exchange resin is styrene-divinylbenzene resin with sulphonic acid groups.

The acetal of 3-pentenal is represented by the following formula:

CH₃-CH=CH-CH₂-CH-(OR)₂ (II)

in which R corresponds with the organic group of the alcohol.

In a subsequent step the hydroformylation reaction mixture (containing 2-, 3- and 4-pentenal) or the acetalisation reaction mixture (containing acetals of 2-, 3- and 4-pentenal) is subjected to one or more separation stages. The hydroformylation reaction product (pentenals and/or monacetals thereof) can be separated from the reaction mixture using any separation technique known to a person skilled in the art. Examples of suitable separation techniques are (vacuum) distillation, simple evaporation under vacuum or stripping with (synthesis) gas. For example, the liquid reaction mixture (containing pentenals, hydroformylation catalyst, solvent, excess ligand and optionally acetals of pentenals and acetalisation catalyst) removed from the hydroformylation reactor is passed to a vaporiser wherein the desired pentenals or acetals thereof are separated from the liquid reaction mixture via distillation in one or more stages, condensed and collected in a product receiver, and further purified if desired. The distillation of the desired pentenals from the liquid hydroformylation mixture is preferably carried out at relatively low temperatures, preferably lower than 150 °C and more preferably at a temperature between 30 and 100 °C. The distillation is preferably carried out at a pressure lower than 20 kPa and more preferably at a pressure lower than 10 kPa.

Before the acetalisation reaction mixture is subjected to distillation, the acetalisation reaction mixture is preferably first subjected to a neutralisation step to make the acetalisation reaction mixture non-acidic. It has been found that omitting such a neutralisation step results in the formation of pentenals from the corresponding monacetals. The pentenals were found to be less stable (chemically and thermically) compared to the corresponding acetals. The neutralisation can be carried out by adding a base to the acetalisation reaction mixture. Examples of suitable bases are sodium hydroxide, potassium hydroxide and ammonium hydroxide. The amount of base which is added for neutralising the acetalisation reaction mixture must be such that the molar ratio of base to acid is larger than 1:1, preferably larger than 2:1. In case the acetalisation reaction is performed in the presence of an acidic ion exchange resin, this resin is preferably removed before the acetalisation reaction mixture is subjected to distillation. In the latter case, the neutralisation step becomes superfluous.

In one embodiment of the invention, the pentenals obtained in the hydroformylation are in a further step oxidized into 3-pentenoic acid and/or ester. When the oxidation (of pentenal(s)) is performed in the presence of an alcohol, the main product will be a pentenoate ester. For example, the oxidation of 3-and/or 4-pentenal carried out in the presence of methanol will produce methyl-3-and/or -4-pentenoate. This embodiment of the process of the invention may also be accomplished in two steps. First, the pentenals can be oxidized to pentenoic acid and subsequently esterified with an alcohol to give alkyl pentenoate esters.

The alcohol used in the oxidation is preferably the same as the alcohol used in the acetalisation.

3-Pentenoic acid or 3-pentenoate ester can be, for example, advantageously used as an intermediate compound in the preparation of caprolactam which is a raw material for the preparation of nylon-6. 3-Pentenoic acid and/or 3-pentenoate ester is for example further subjected to a hydroformylation reaction as for example described in WO-A-9733854, the complete disclosure of which is incorporated as reference.

In another and more preferred embodiment of the invention, the monacetals of pentenals obtained in the acetalisation are in a further step oxidized into 3-pentenoate ester.

It has been found that the preparation of pentenoic acid and/or pentenoate ester starting from butadiene by hydroformylation of butadiene to pentenal(s) and/or acetals thereof in a first step and subsequent oxidation of the thus obtained pentenal(s) and/or acetal(s) is advantageous in that this process can be conducted in the substantial absence of either an acid or any halogen or halogenide compound. Halogen or halogenide compounds are often used as promotors for the direct carbonylation of compounds such as butadiene. Acids may also be used in alternative processes for the direct carbonylaiton of butadiene. An advantage of the two step route (hydroformylation followed by oxidation) is that acids and halogen or halogenide promotor compounds are corrosive and their reduction and/or elimination is desirable to improve the operation and safety of the process. The two step route based on pentenal(s) and/or acetals thereof is completely acid and halogen or halogenide free.

The oxidation is accomplished by adding oxygen to the mixture containing pentenals and /or acetals thereof. The oxygen used for the production of pentenoic acid and/or pentenoate ester is molecular oxygen, which may be in the form of oxygen gas or a gas mixture of an oxygen gas and an inert diluent gas, such as nitrogen gas and carbon dioxide gas. Molecular oxygen may be provided in the form of air. The amount of oxygen needed is not less than the stoichiometric quantity required for the reaction. Generally, the amount of oxygen is controlled such that the oxygen concentration in the gaseous effluent is less than 5 vol.%.

The oxidation is preferably carried out in the presence of an oxidation catalyst. Suitable oxidation catalysts and method for their preparation are for example described in EP-A-857512, EP-A-199530 and US-A-4518796, the complete disclosures are incorporated as references. An exemplary oxidation catalyst is a SiO₂ carrier having supported thereon Pd and Pb as described in EP-A-857512. It has been found that treating this catalyst with base or hot water results in a further increase of the activity of the catalyst. Another exemplary oxidation catalyst, disclosed in for example EP-A-199530, is the catalyst with general formula PdₐTe_{b}Zn_{d}Eₑ, where E is one or more metals from the group consisting of group IA, IIA, IVA, VIIB or VIII metals, As or Sb and a, b, d and e are from about 0 to 3 with the proviso that at least d or e are different from zero.

The oxidation of the acetals of pentenals is preferably carried out in the presence of water and a hydrolysis catalyst, for example nonanoic acid. It has been found that the selectivity to 3-pentenoate ester is increased when water is added after having treated the acetal with the hydrolysis catalyst for a certain amount of time.

The oxidation may be performed in for example a slurry reactor or a packed bed reactor. After the oxidation, the pentenoic acid and/or ester thereof can be isolated by for example distillation.

In another embodiment of the invention, the pentenals obtained in the hydroformylation reaction or preferably the acetals of the pentenals obtained in the acetalisation reaction are further subjected to a carbonylation reaction in the presence of a carbonylation catalyst to obtain 5-formylvalerate and/or the acetals of 5-formylvalerate. The carbonylation is preferably performed in the presence of an alcohol. Suitable carbonylation catalysts are described in for example WO-A-9702091 and EP-A-577205. Suitable carbonylation conditions are those conditions which are applied for the carbonylation of monoalkene compounds. The 5-formylvalerate and/or the acetals of 5-formylvalerate is advantageously further subjected to a hydrogenation reaction in the presence of ammonia, hydrogen and a hydrogenation catalyst (reductive amination) to obtain the corresponding 6-aminocaproate. Suitable hydrogenation catalysts and reductive amination conditions are described in for example EP-A-729944, EP-A-729943, WO-A-0014062.

In another embodiment of the invention, the pentenals obtained in the hydroformylation reaction or preferably the acetals of the pentenals obtained in the acetalisation reaction are further subjected to a hydrogenation reaction in the presence of ammonia, hydrogen and a hydrogenation catalyst to a reaction mixture which is subsequently subjected to a carbonylation reaction. The hydrogenation catalyst is selected from catalysts which selectively reduce the intermediate imine bond.

The invention is further described in the following non-limiting examples:

### Example I

Example I illustrates the synthesis of pentenals and their purification.

A solution containing 0.566 mmol Rh(AcAc)(CO)₂ (AcAc=acetylacetonate), 0.677 mmol dppf (dppf=diphenyl-phosphinoferrocene), 2.9335 gram nonane (GC internal standard) in 154.8 gram xylene was prepared in a drybox under an atmosphere of nitrogen. This solution was loaded into an autoclave which was purged with CO/H₂. The autoclave was closed and flushed with CO/H₂. The autoclave was heated to 100°C while stirring at 1000 rpm and 25.45 gram butadiene was injected into the autoclave. The pressure was adjusted to 6 MPa by adding CO/H₂. After 120 minutes, a yield of 15.43 g linear 3- and 4-pentenal (39% on butadiene) and 1.67 g (4% on butadiene) linear pentanal was found. Furthermore a yield of 8.14 g (15% on butadiene) C₆-dialdehydes were found.

The reaction mixture was distilled batchwise under 7 kPa in a SPALT-column. The bottom temperature was approximately 70 °C, the top temperature was approximately 37 °C. The first cut contained 79 wt% linear 3-and 4-pentenal and 18% linear pentanal. No butadiene dimers such as VCH, cyclooctadiene and octatriene were detected in this first cut.

### Example II

Example II illustrates that pentenals are relatively stable under strictly neutral conditions.

The purified fraction of 3- and 4-pentenal of example I was stored for one week at room temperature under a nitrogen atmosphere. Analysis before and after this week of storage showed that the pentenal concentration had dropped only by 2.2 wt%. The main degradation products were analysed to be the aldolcondensates of 3- and 4-pentenal.

Another batch of pentenals, prepared by organic synthesis, was contacted with the base triethylamine. Upon contacting with triethylamine, more than 80 % of the pentenals directly converted to heavier products such as aldolcondensates.

### Example III

Example III illustrates that pentenal can be oxidatively esterfied to methyl-3-pentenoate.

A sample of a solution containing 1 gram of pentenals (80.5% 3-pentenal, 2.7% 4-pentenal and 17.1% 2-pentenal), 10 grams of methanol and 0.5 gram of orthodichlorobenzene (GC internal standard) was heated at 50 °C under 0.7 MPa of air for 1 hour in the presence of 10% by weight of a solid catalyst (Example 3A: Pd₄TeZnPb; Example 3B: Pd₄TeZnPbBi; Example 3C: Pd₄TeZn) prepared according to EP-A-199530. The products were analysed by Gas Chromatography with a Restex^{R} (from Restek Corporation Benner Circle, USA) amine column (15m*0.25mm). The conversions are shown in Table 1.

**Table 1**

| **Catalyst** | **Pd**_{**4**}**TeZnPb** | **Pd**_{**4**}**TeZnPbBi** | **Pd**_{**4**}**TeZn** |
|---|---|---|---|
| PAL¹ conversion² | 67.7 | 60.4 | 48.0 |
| MP³ selectivity⁴ | 75.9 | 54.1 | 62.5 |

| | | | |
|---|---|---|---|
| ¹PAL=pentenal | | | |
| ²PAL conversion=100*(4PAL+3PAL+2PAL) /(4PAL+3PAL+2PAL) ₜ₌₀ | | | |
| ³MP=methyl pentenoate | | | |
| ⁴MP selectivity= 100*(4MP+3MP+2MP)/[(4PAL+3PAL+2PAL) ₜ₌₀-(4PAL+3PAL+2PAL)] | | | |

### Example IV

Example IV illustrates the synthesis of the acetals of pentenals and their purification.

A solution containing 0.573 mmol Rh(AcAc)(CO)₂, 0.677 mmol dppf (diphenyl-phosphinoferrocene), 3.771 gram anisol (GC intemal standard), 0.10 g paratoluene sulphonic acid in 142.2 gram MeOH was prepared in a drybox under an atmosphere of nitrogen. This solution was loaded into an autoclave which was purged with CO/H₂. The autoclave was closed and flushed with CO/H₂. The autoclave was heated to 100°C while stirring at 1000 rpm and 54.54 gram butadiene was injected into the autoclave. The pressure was adjusted to 5 MPa by adding CO/H₂. After 120 minutes, a yield of 39.14 g linear acetals of pentenal (30% on butadiene) and 2.59 g (2% on butadiene) linear acetal of pentanal was found. Furthermore, a yield of 2.5 g (3% on butadiene) 3- and 4-pentenal were found.

The reaction mixture was neutralised with excess sodium hydroxide. Then, the reaction mixture was distilled batchwise in a SPALT-column. First, MeOH was removed at 0.1 MPa with the bottom- and toptemperature increasing from 74 to 106 °C and 64 to 91 °C, respectively. The reflux ratio was 2. Then the pressure was reduced to 7 kPa and the reflux ratio increased to 5 and two more cuts were collected with increasing purity of 77 to 84 wt% of acetals of pentenal. The reflux ratio was further increased to 10 and three more cuts were collected with increasing purity of 90-98 % acetals of 3- and 4-pentenal. The top- and bottom temperature increased from 88 to 112 °C and 36 to 72 °C, respectively, during collection of the five cuts. The total yield of acetals of 3- and 4-pentenal in the five cuts was 93% of the feed. No butadiene dimers such as VCH, cyclooctadiene and octatriene nor MeOH were detected in the five cuts.

A slurry containing 3.58 g acetals of 3- and 4-pentenal, 4 wt% (on total reaction mixture) PdPb/SiO₂ catalyst, 0.025 g nonanoic acid, 1.170 gram anisol (GC internal standard) in 36.1 gram MeOH was charged to an autoclave. The autoclave was closed and flushed with 5 vol% O₂ in N₂. The autoclave was heated to 80°C while stirring at 1000 rpm and 1 Nlitre/hr of 5 vol% O₂ in N₂ was passed through the reactor. The pressure was adjusted to 0.5 MPa. After 2600 minutes, a yield of 19% of methyl-3-pentenoate was found.

### Example V

Example V illustrates that considerable retroreaction occurs when the preneutralisation is skipped. It also demonstrates the bench scale preparation of acetals of 3- and 4 pentenal with good yields and reaction rates.

Four batches (HDL-1 through HDL-4) were run for bench scale preparation of acetals of 3- and 4-pentenal. The reaction conditions employed were the following: a temperature of 100 °C, a pressure of 7 MPa, a CO/H₂-ratio of 1:1 mole/mole and a stirring speed of 850 rpm.

The chemicals and reaction rates employed are listed in table 2 below:

**Table 2**

| | **HDL-1** | **HDL-2** | **HDL-3** | **HDL4** |
|---|---|---|---|---|
| **COMPONENT** | | | | |
| MeOH (g) | 868 | 910 | 853 | 910 |
| Rh(AcAc)(CO)₂ (g) | 1.007 | 1.0305 | 1.0022 | 1.0153 |
| Diphenylphosphinoferrocene (g) | 2.6265 | 2.6178 | 2.5966 | 2.6059 |
| p-toluenesulfonic acid (g) | 0.678 | 0.632 | 0.725 | 0.838 |
| Butadiene (g) | 170 | 167 | 162 | 173 |
| Total (g) | 1042 | 1081 | 1019 | 1087 |
| **Initial butadiene concentration** (wt%) | 16.3 | 15.4 | 15.9 | 15.9 |
| **Initial rhodium (Rh) concentration** (ppmw) | 385.3 | 380.1 | 392.1 | 372.4 |
| **Rh/ligand ratio** (mole/mole) | 1.21 | 1.18 | 1.21 | 1.20 |
| **Reaction rates** | | | | |
| Total gas consumption(*) | 130 | 139 | 128 | 132 |
| Reaction time | 1.5 | 1.75 | 1.75 | 1.5 |
| Average reaction rate | 1.38 | 1.27 | 1.17 | 1.40 |
| Initial reaction rate large than | 2.68 | 2.68 | 2.68 | 2.68 |

| | | | | |
|---|---|---|---|---|
| (*) Gas consuption is only indicative, because the flow exceeded The maximum range of the flow indicator during the first 30 min. | | | | |

Analysis showed the following selectivities on butadiene at approximately 90 % butadiene conversion (table 3):

**Table 3**

| | Selectivity (mole%) |
|---|---|
| Acetals of 3- and 4 pentenal | 69.4 |
| 3- and 4-pentenal | 8.5 |
| Total of 3- and 4-pentenal + acetals thereof | 78.0 |
| | |
| Pentanal | 0.4 |
| Acetal of pentanal | 3.2 |
| C₆-dialdehydes | 0.1 |
| Diacetal of dialdehydes | 13.9 |
| Other heavies | 4.4 |
| | |
| Total | 100.0 |

The batches were combined for destillation *without* preneutralisation. The combined four batches were distilled in a column comprising 1.5 m Sulzer BX packing and a wiped film evaporator as reboiler. Using a reflux ratio of 3, MeOH was removed at atmospheric pressure with a temperature of 78 °C at the bottom end of the packing and 64 °C at the top end of the packing. After removal of MeOH, the column pressure was decreased to 7 kPa and the reflux ratio was reduced to 1. Initially 138 grams of a heterogeneous azeotrope of organics with water was distilled off at a top temperature of 30-32 °C. After this, a fraction of 717 g was collected at a top temperature of 59 °C with the following composition:

| | |
|---|---|
| 3- and 4-pentenal | 13,2 wt% |
| Acetals of 3- and 4-pentenal | 77,4 wt% |
| MeOH | 2,9 wt% |
| Acetal of pentanal | 3,5 wt% |
| Pentanal | 0,9 wt% |
| Others | 2,1 wt% |
| Total | 100 wt% |

The fact that a considerable amount of MeOH is found in this fraction can be explained by the retroreaction of acetals to aldehyde and methanol in the presence of the paratoluene sulphonic acid. This retroreaction can be prevented by preneutralisation as shown in example IV.

### Example VI

Example VI illustrates the preparation of dimethylacetales of formylvalerate by carbonylation of dimethylacetale of 3-pentenal.

A solution containing 0.24 mmole Pd(acetate)₂, 1.15 mmole of 1,4-bis(diphenyl)phosphinobutane. 2.29 g p-toluene sulfonic acid, and 0.8 g nonane (GC internal standard) in 50 g anisole was prepared in a drybox under an atmosphere of nitrogen. This solution was loaded in a 150 ml autoclave which was purged with CO. The autoclave was closed and flushed with 3 MPa CO. The reactor was heated to 120 °C, while stirring at 1000 rpm, and 7.5 g of the dimetyl acetale of 3-pentenal, and 3.1 g of methanol was injected via an ampoule. The pressure was adjusted to 6 MPa CO. Samples were taken periodically for gas chromatographic product analysis.
GC analysis after 60 minutes indicated a yield of 20% to the dimethyl acetales of formylvalerates of which 12 % was assigned to the dimethyl acetale of 5-formylvalerate.

### Example VII

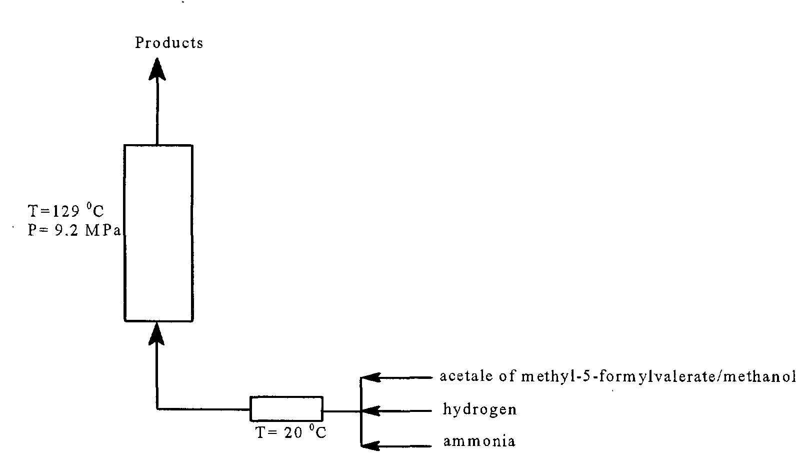

Example VII was performed in a unit as schematically shown in the figure.

A solution containing 10 wt% acetal of methyl-5-formylvalerate in methanol was continuously fed to a 5 cc reactor (length 10 cm, diameter 0.4 cm) with a rate of 219 ml/hour and mixed with liquid ammonia (70 g/hour), and hydrogen (28 liter/hour). The temperature was kept at 20 °C. The resulting mixture was continuously passed through a column containing Raney Nickel catalyst at 129 °C and 10 MPa pressure. The product stream was brought to ambient pressure and temperature and analysed by gas chromatographic techniques. Analysis indicated after 1 hour a conversion of 96% and selectivity of 95% to methyl-6-aminocaproate.

## Claims

1. Process for the preparation of a caprolactam precursor from butadiene, **characterised in that**, the process comprises a step in which butadiene is hydroformylated into a mixture of pentenals in the presence of carbon monoxide, hydrogen and a hydroformylation catalyst.

2. Process according to claim 1, **characterised in that**, the process further comprises the step of oxidizing the pentenals.

3. Process according to claim 2, **characterised in that**, the oxidation is performed in the presence of an oxidation catalyst.

4. Process according to claim 2 or 3, **characterised in that**, the oxidized pentenal reaction mixture comprises pentenoic acid.

5. Process according to claim 2 or 3, **characterised in that**, the oxidation is performed in the presence of an alcohol.

6. Process according to claim 5, **characterised in that**, the oxidized pentenal reaction mixture comprises pentenoate esters.

7. Process according to any one of claims 1-6, **characterised in that**, the pentenals are prepared and treated under strictly neutral conditions.

8. Process according to claim 1, **characterised in that**, the hydroformylation is performed in the presence of an alcohol resulting in a reaction mixture comprising an acetal of pentenals.

9. Process according to claim 8, **characterised in that**, the hydroformylation is performed in the presence of an acidic acetalisation catalyst.

10. Process according to claim 9, **characterised in that**, the reaction mixture comprising the acetal of pentenals is neutralized before separating the acetal from the reaction mixture.

11. Process according to anyone of claims 8-10, **characterised in that**, the process further comprises the step of oxidizing the monacetal of pentenals.

12. Process according to claim 11, **characterised in that**, the oxidation is performed in the presence of an oxidation catalyst.

13. Process according to claim 12, **characterised in that**, the oxidation is performed in the presence of water and an acid.

14. Process according to anyone of claims 11-13, **characterised in that**, the oxidized acetal reaction mixture comprises pentenoate ester.

15. Process according to claim 1 or 8, **characterised in that**, the process further comprises the step of carbonylating the pentenals and/or acetals thereof resulting in a reaction mixture comprising the acetal of a 5-formylvalerate.

16. Process according to claim 15, **characterised in that**, the acetal of 5-formylvalerate is reacted with ammonia and hydrogen in the presence of a hydrogenation catalyst into the corresponding 6-aminocaproate.
